(19) Europäisches Patentamt
European Patent Office
Office européen des brevets

(11) Veröffentlichungsnummer: **0 613 471 B1**

# (12) EUROPÄISCHE PATENTSCHRIFT

(45) Veröffentlichungstag der Patentschrift: **14.06.95**

(51) Int. Cl.$^6$: **C07D 251/70**, A01N 43/68

(21) Anmeldenummer: **92919871.1**

(22) Anmeldetag: **29.09.92**

(86) Internationale Anmeldenummer:
**PCT/EP92/02249**

(87) Internationale Veröffentlichungsnummer:
**WO 93/10105 (27.05.93 93/13)**

## (54) 1,3,5-TRIAZIN-2,4,6-TRIS-ALKYLAMINOCARBONSÄUREAMINOESTER, DIESE ENTHALTENDE BIOZIDE MITTEL SOWIE VERFAHREN ZU IHRER HERSTELLUNG.

(30) Priorität: **19.11.91 DE 4138089**

(43) Veröffentlichungstag der Anmeldung:
**07.09.94 Patentblatt 94/36**

(45) Bekanntmachung des Hinweises auf die Patenterteilung:
**14.06.95 Patentblatt 95/24**

(84) Benannte Vertragsstaaten:
**AT BE CH DE DK ES FR GB GR IE IT LI LU MC NL SE**

(56) Entgegenhaltungen:
**EP-A- 0 046 139**
**EP-A- 0 262 086**

**CHEMICAL ABSTRACTS, vol. 60, no. 4, 17. Februar 1964, Columbus, Ohio, US;abstract no. 4145e, H. NESTLER ET AL. 'Preparation of N-(1,3,5-triazinyl)amino acid derivatives.' Spalte 4145 ; & JOURNAL FUER PRAKTISCHE CHEMIE vol. 23, No. 3-4, 1963, LEIPZIG pages 173-185**

**CHEMICAL ABSTRACTS. REGISTRY HANDBOOK - NUMBER SECTION. 1982, COLUMBUS US Seite156**

(73) Patentinhaber: **CG-CHEMIE GMBH**
**Billbrookdeich 157**
**W-2000 Hamburg 74 (DE)**

(72) Erfinder: **LESMANN, Jörg**
**Fockenweide 33**
**D-2050 Hamburg 80 (DE)**
Erfinder: **SCHÄFER, Hermann, Georg**
**Hikeberg 18**
**D-2000 Hamburg 70 (DE)**

(74) Vertreter: **Glawe, Delfs, Moll & Partner**
**Patentanwälte**
**Postfach 26 01 62**
**D-80058 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft 1,3,5-Triazin-2,4,6-tris-alkylaminocarbonsäureaminoester, diese enthaltende biozide bzw. biostatische Mittel und Verfahren zu ihrer Herstellung. Die vorgenannten Verbindungen werden im folgenden als "Aminoester der Erfindung" bezeichnet.

Die den Aminoestern der Erfindung zugrundeliegenden Triazintricarbonsäuren, d.h. die 2,4,6-Tris-(omega'-carboxyalkylamino)-1,3,5-triazine, im folgenden 1,3,5-Triazin-2,4,6-tris-alkylaminocarbonsäuren genannt, sind in J. Prakt. Chemie, 23 (1963), S. 173 bis 185, sowie in der EP-B 0 046 139 beschrieben. Die EP-B 0 046 139 betrifft weiterhin die Verwendung der genannten Triazintricarbonsäuren sowie deren Alkalimetall-, Mono-, Di- oder Triethanolammonium-Salze als Korrosionsinhibitoren in wäßrigen Systemen. Die EP-B 0 046 139 beschreibt weiterhin die Mono-, Di- und Triethanolammoniumsalze dieser Triazintricarbonsäuren, die als Korrosionsinhibitoren in wäßrigen Systemen eingesetzt werden können; eine analoge Anwendung dieser Verbindungen in wäßrigen Systemen, z.B. Kühlflüssigkeiten, Kühlschmierstoffen, Anstrichstoffen oder Reinigern, ist in der EP-A 0 262 086 offenbart.

Wäßrigen Systemen der vorgenannten Art müssen zur Verhinderung eines Befalls mit Bakterien, Hefen und/oder Pilzen biozide bzw. biostatische Mittel zugesetzt werden. Als hierfür geeignete Mittel wurden bisher halogenhaltige Verbindungen sowie z.B. Borsäure und Umsetzungsprodukte von Borsäure mit Alkanolaminen verwendet, siehe Ullmanns Encyklopädie der technischen Chemie, 4. Auflage, Band 8, Verlag Chemie, Weinheim 1974, S. 653-655. In anderen Fällen wurden Formaldehyd oder Formaldehyd-Derivate als Biozid zugesetzt. Halogenhaltige Verbindungen, Borsäure und Borsäurederivate sowie Formaldehyd und dessen Derivate sind jedoch aus verschiedenen Gründen unerwünscht. Daher besteht ein zunehmender Bedarf an bioziden Mitteln zur Verwendung in wasserhaltigen Systemen, die frei von halogenhaltigen Verbindungen, Formaldehyd, Formaldehyd-Derivaten, Borsäure oder Borsäurederivaten sind.

Es wurde nun gefunden, daß die Aminoester der Erfindung bei ihrem Einsatz in wäßrigen Systemen der vorgenannten Art bereits in geringen Konzentrationen ausgezeichnete biozide bzw. biostatische Eigenschaften entfalten.

Demgemäß betrifft die Erfindung 1,3,5-Triazin-2,4,6-trisalkylaminocarbonsäureaminoester der allgemeinen Formel

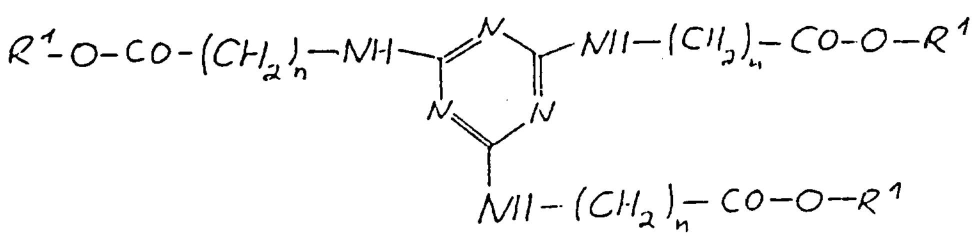

(I)

in der
n eine Zahl im Bereich von 4 bis 11 bedeutet und
$R^1$ einen Rest eines Alkanolamins der allgemeinen Formel

$(R^2)_3N$ (II)

in der
mindestens eine der Gruppen $R^2$

a) eine Hydroxyalkylgruppe mit 2 bis 4 Kohlenstoffatomen,
b) eine Hydroxyalkyl-oxyalkylengruppe mit 4 bis 6 Kohlenstoffatomen oder
c) eine Dihydroxyalkylgruppe mit 3 bis 6 Kohlenstoffatomen

und, wenn weniger als drei der Gruppen $R^2$ die vorstehende Bedeutung aufweisen, die übrigen Gruppen $R^2$ Wasserstoff sind,
bedeutet.

Eine bevorzugte Ausführungsform der Erfindung betrifft Aminoester der allgemeinen Formel I, in der n die Zahl 5 bedeutet.

Die Alkanolamine der allgemeinen Formel II weisen primäre, sekundäre oder tertiäre Amino- und freie Hydroxylgruppen auf. Bei der Umsetzung von primäre oder sekundäre Aminogruppen aufweisenden

Alkanolaminen mit Carbonsäuren können sowohl Amide als auch Ester entstehen, die miteinander im Gleichgewicht stehen, siehe "Surfactants in Consumer Products", Hrsg. J. Falbe, Springer-Verlag, Heidelberg 1987, S. 96. Aus Gründen der Übersichtlichkeit sind die Umsetzungsprodukte von 1,3,5-Triazin-2,4,6-tris-alkylaminocarbonsäuren mit Alkanolaminen zu Verbindungen der allgemeinen Formel I, in der $R^1$ der Rest eines Alkanolamins der allgemeinen Formel II ist, hier nur als Aminoester dargestellt. Es ist für den Fachmann aber ohne weiteres ersichtlich, daß unter die so definierten 1,3,5-Triazin-2,4,6-tris-alkylcarbonsäurederivate auch die entsprechenden Alkanolamide fallen.

Typische Beispiele für Hydroxyalkylgruppen mit 2 bis 4 Kohlenstoffatomen, die die Gruppe $R^2$ bilden können, sind 2-Hydroxyethyl-, 1-Methyl-2-Hydroxyethyl-, 2-Hydroxypropyl-, 3-Hydroxypropyl-, 2-Hydroxybutyl-, 4-Hydroxybutyl- und 2-Methyl-2-hydroxypropylgruppen; für Hydroxyalkyl-oxyalkylengruppen mit jeweils 2 bis 4 Kohlenstoffatomen in dem Hydroxyalkyl- und Oxyalkylenrest, Hydroxyethyl-oxyethylen-, Hydroxypropyl-oxyethylen-, Hydroxyethyl-diethylenoxy-, Hydroxyethyl-oxypropylen- und Hydroxypropyl-oxypropylengruppen und für Dihydroxyalkylgruppen mit 3 bis 6 Kohlenstoffatomen 2,3-Dihydroxypropyl-, 3,4-Dihydroxybutyl-, 1,3-Dihydroxypropyl- und 1,3-Dihydroxy-2-methyl- oder -ethyl-propylgruppen; weiterhin auch Hydroxyethyl-, Hydroxypropyl- und Hydroxybutyl-oxybutylengruppen.

Verbindungen der allgemeinen Formel I, bei denen $R^1$ einen Rest eines Alkanolamins der allgemeinen Formel II bedeutet, sind durch Umsetzung von 1,3,5-Triazin-2,4,6-tris-alkylaminocarbonsäuren der allgemeinen Formel

1,3,5-Triazin-2,4,6-tris[-NH-$(CH_2)_n$-COOH]      (III)

in der n wie oben definiert ist,
mit Alkanolaminen der allgemeinen Formel II nach an sich bekannten Verfahren erhältlich.

Für viele Anwendungszwecke ist es nicht erforderlich, daß die Aminoester der Erfindung in Substanz isoliert werden. Es reicht vielmehr aus, wenn man die Aminoester der Erfindung "in situ", z.B. in einem Überschuß der Alkanolamine der allgemeinen Formel II, herstellt und gegebenenfalls den Überschuß der Alkanolamine mit geeigneten Säuren, die den angestrebten Verwendungszweck nicht stören oder unter Umständen sogar fördern, neutralisiert.

Bevorzugt ist daher die Umsetzung mit einem, bezogen auf die 1,3,5-Triazin-2,4,6-tris-alkylaminocarbonsäuren, molaren Überschuß der Alkanolamine. Der nicht umgesetzte Teil der Alkanolamine kann zur Einstellung eines pH-Wertes von 4,5 bis 9,5 mit organischen Säuren, ausgewählt aus der von geradkettigen oder verzweigten, gesättigten oder ungesättigten Fettsäuren mit 5 bis 22 Kohlenstoffatomen gebildeten Gruppe, umgesetzt werden. Beispiele für die genannten Fettsäuren sind Pentansäure, Hexansäure, Heptansäure, Octansäure, Nonansäure, Decansäure, Undecansäure, Dodecansäure, Tridecansäure, Tetradecansäure, Pentadecansäure, Hexadecansäure, Heptadecansäure, Octadecansäure, Nonadecansäure, Eicosansäure, Heneicosansäure, Docosansäure, 10-Undecensäure, 9c-Dodecensäure, 9c-Tetradecensäure, 9c-Hexadecensäure, 6c-Octadecensäure, 6t-Octadecensäure, 9c-Octadecensäure, 9t-Octadecensäure, 9c,12c-Oc-tadecadiensäure, 9t,12t-Octadecadiensäure, 9c,12c,15c-Octadecatriensäure, 9c,11t,13t-Octadecatriensäure, 9c-Eicosensäure, 5,8,11,14-Eicosatetraensäure, 13c-Docosensäure, 13t-Docosensäure, 4,8,12,15,19-Docosapentaensäure, 12-Hydroxy-Octadecansäure und 12-Hydroxy-9c-octadecensäure, wobei c eine cis-Doppelbindung und t eine trans-Doppelbindung anzeigt, sowie technische Gemische derselben. Besonders geeignet sind weiterhin Fettsäuren bzw. Fettsäuregemische, die aus nachwachsenden Rohstoffen, insbesondere pflanzlichen und/oder tierischen Fetten und Ölen, erhältlich sind, z.B. Capron-, Capryl-, Caprin-, Laurin-, Myristin-, Palmitin-, Stearin-, Öl-, Ricinol-, Linol-, Eruca- und Behensäure.

Bevorzugt wird der nicht umgesetzte Teil der Alkanolamine mit geradkettigen oder verzweigten, gesättigten oder ungesättigten Fettsäuren mit 5 bis 11 Kohlenstoffatomen umgesetzt. Wenn man auf diese Weise keine stabilen Lösungen oder Emulsionen erhält, können zur Einstellung der gewünschten Hydrophil/Hydrophob-Balance zusätzlich geradkettige oder verzweigte, gesättigte oder ungesättigte Fettsäuren mit 12 bis 22 Kohlenstoffatomen verwendet werden.

Bevorzugt sind solche Aminoester der allgemeinen Formel I, die keine sekundären oder tertiären Aminofunktionen enthalten. Sekundäre Alkanolamine können mit Nitritionen unerwünschte, stabile Nitrosamine bilden. Tertiäre Alkanolamine können unter Umständen durch Dealkylierung sekundäre Alkanolamine bilden. Dagegen bilden primäre Alkanolamine in der Regel keine stabilen Nitrosamine, sondern dienen vielmehr wegen des schnellen Zerfalls der intermediär gebildeten Nitrosamine als Abfänger für Nitritionen. Wenn man dennoch von sekundären Alkanolaminen abgeleitete Aminoester der allgemeinen Formel I einsetzen will, ist die Verwendung eines Gemisches von von primären und sekundären Alkanolaminen abgeleiteten Verbindungen bevorzugt, da dann die Bildung der instabilen primären Nitrosamine schneller erfolgt als die der sekundären Nitrosamine.

Somit betrifft die Erfindung gemäß einem weiteren Aspekt Aminoester der allgemeinen Formel I, die frei von sekundären oder tertiären Aminofunktionen sind und somit keine stabilen Nitrosoverbindungen bilden können bzw. die bei gleichzeitiger Anwesenheit der analogen sekundäre oder tertiäre Aminofunktionen enthaltenden Verbindungen der allgemeinen Formel I die Ausbildung stabiler Nitrosoverbindungen verhindern.

Mit dem Verfahren der Erfindung können auch biozide bzw. biostatische Gemische von Monocarbonsäurealkanolamiden und Aminoestern der allgemeinen Formel I sowie ggf. Alkanolammoniumsalzen der Monocarbonsäuren und/oder der 1,3,5-Triazin-2,4,6-tris-alkylaminocarbonsäuren hergestellt werden.

Die vorgenannten bioziden bzw. biostatischen Gemische können durch Vermischung der Einzelkomponenten hergestellt werden. Zweckmäßigerweise werden sie jedoch hergestellt, in dem man die Alkanolamide in situ aus den Monocarbonsäuren und den 1,3,5-Triazin-tris-alkylaminocarbonsäuren der allgemeinen Formel III, in der n wie oben definiert ist, mit Alkanolaminen der allgemeinen Formel II, in der $R^2$ wie oben definiert ist, vorzugsweise in einem Überschuß der Alkanolamine, herstellt.

Vorzugsweise werden primäre Alkanolamine oder Gemische aus primären und sekundären Alkanolaminen verwendet.

Bevorzugt werden pro Mol der 1,3,5-Triazin-2,4,6-tris-alkylaminocarbonsäuren10 bis 50 Mol, insbesondere 10 bis 30 Mol, der Alkanolamine der allgemeinen Formel II und 0,5 bis 5 Mol der Monocarbonsäuren umgesetzt.

Die Herstellung der Aminoester der Erfindung erfolgt bei einer Temperatur im Bereich von 100 bis 180, insbesondere 130 bis 180 °C.

Als Monocarbonsäuren werden bevorzugt geradkettige oder verzweigte, gesättigte oder ungesättigten Fettsäuren mit 3 bis 22, insbesondere 12 bis 22 Kohlenstoffatomen, verwendet, die in einer ersten Stufe mit den Alkanolaminen zu den entsprechenden Aminoestern bzw. Alkanolamiden umgesetzt werden, gefolgt von einer Zugabe und Umsetzung der 1,3,5-Triazin-2,4,6-tris-alkylaminocarbonsäuren mit dem vorhandenen Überschuß an Alkanolaminen zu den Aminoestern der Erfindung in einer zweiten Stufe. Diese Umsetzung kann auch in einer anderen Reihenfolge oder in einer einzigen Stufe durchgeführt werden, wobei dann aber unter Umständen weniger ausgeprägte biozide bzw. biostatische Eigenschaften des Gemisches erhalten werden.

Weiterhin werden als Monocarbonsäuren bevorzugt Ethercarbonsäuren der allgemeinen Formel

$R^3$-(O-$C_mH_{2m}$)$_q$-O-$CH_2$-COOH (III)

in der

- $R^3$ eine geradkettige oder verzweigte Alkyl- oder Alkenylgruppe mit 9 bis 18 Kohlenstoffatomen,
- m die Zahl 2 und/oder 3 und
- q eine Zahl im Bereich von 0 bis 100, vorzugsweise von 0 bis 20, bedeuten,

verwendet. Die Umsetzung kann hier in beliebiger Reihenfolge, aber auch in einer einzigen Stufe erfolgen.

Ebenso werden als Monocarbonsäuren bevorzugt Arylsulfonamidocarbonsäuren der allgemeinen Formel

($R^4$)Aryl-$SO_2$-N($R^5$)-$R^6$-COOH (Va)

in der

$R^4$ Wasserstoff oder eine Methyl- oder Ethylgruppe oder mehrere, $R^5$ Wasserstoff, eine Methyl-, Ethyl-, beta-Cyanoethyl- oder Hydroxymethylgruppe, $R^6$ eine Alkylengruppe mit 4 bis 6 Kohlenstoffatomen und Aryl einen Phenyl-, Naphthyl- oder Anthracenylrest bedeuten, Alkylsulfonamidocarbonsäuren der allgemeinen Formel

$R^7$-$SO_2$-$NR^8$-$CH_2$-COOH (Vb)

in der $R^7$ eine geradkettige oder verzweigte Alkylgruppe mit 12 bis 22 Kohlenstoffatomen und $R^8$ Wasserstoff oder die Gruppe -$CH_2$-COOH bedeuten,
und/oder Halbester bzw. Halbamide der allgemeinen Formel Vc

$R^9$-OOC-$R^{10}$-COOH (Vc)

in der

$R^9$ der Rest eines Alkanolamins der allgemeinen Formel II und

$R^{10}$ ein o-Phenylen-, Vinylen- oder 1,2-Ethylenrest ist,

verwendet. Auch hier kann die Umsetzung in beliebiger Reihenfolge, aber auch in einer einzigen Stufe durchgeführt werden.

Es konnte noch nicht festgestellt werden, ob die Sulfonamidocarbonsäuren der allgemeinen Formel Va oder Vb mit den Alkanolaminen der allgemeinen Formel II zu Sulfonamidocarbonsäureaminoalkylestern, zu Sulfonamidocarbonsäurealkanolamiden oder zu Gemischen derselben umgesetzt werden. Diese Umsetzungsprodukte werden hier der Einfachheit halber immer als Alkanolamide bezeichnet. Die vorgenannten Sulfonamidocarbonsäuren sind z.B. aus der DEC-C 28 40 112 und der DE-A 33 04 164 bekannt.

Anschließend kann dann in dem erhaltenen Reaktionsgemisch enthaltenes, überschüssiges Alkanolamin zur Einstellung eines pH-Wertes im Bereich von 4,5 bis 9,5 mit Fettsäuren mit 3 bis 22, vorzugsweise 3 bis 11 Kohlenstoffatomen, Ethercarbonsäuren der allgemeinen Formel IV, in der $R^3$, m und q wie oben definiert sind, und/oder Aryl- bzw. Alkylsulfonamidocarbonsäuren der allgemeinen Formel Va bzw. Vb, in denen $R^4$, $R^5$, $R^6$, $R^7$, $R^8$ und $R^9$ wie oben definiert sind, umgesetzt werden.

Es ist bevorzugt, alle Umsetzungen so durchzuführen, daß man das Reaktionsgemisch durchgehend flüssig hält. Man erreicht dies z.B. mit dem bevorzugten, großen Überschuß an Alkanolaminen.

Schließlich können dem Reaktionsgemisch nach der Umsetzung noch die weiter unten beschriebenenen Fungizide, vorzugsweise in einer Menge von 1 Gewichtsteil Fungizide auf 10 bis 100 Gewichtsteile der in dem bioziden bzw. biostatischen Gemisch enthaltenen Aminoester der allgemeinen Formel I, in der $R^1$ und n wie oben definiert sind, zugesetzt werden.

Ein nach der vorstehend erläuterten Umsetzung vorhandener Überschuß an Alkanolaminen wird, wie oben angegeben, zur Einstellung eines geeigneten pH-Bereichs und unter Bildung weiterer Anteile an Alkanolamiden bzw. Alkanolammoniumsalzen vollständig oder teilweise neutralisiert.

Beispiele für geradkettige oder verzweigte, gesättigte oder ungesättigte Fettsäuren mit 3 bis 22 Kohlenstoffatomen sind Propansäure, die o.g. Fettsäuren mit 5 bis 22 Kohlenstoffatomen sowie technische Gemische derselben. Die Umsetzungsprodukte der Alkanolamine mit den Monocarbonsäuren können weiterhin in den wasserhaltigen Systemen als Korrosionsschutzmittel dienen.

Bevorzugte Beispiele für erfindungsgemäß verwendbare Alkanolamine der allgemeinen Formel II, in der $R^2$ wie oben definiert ist, sind Mono-, Di- und Triethanolamin, Mono-, Di- und Tripropanolamin, Mono-, Di- und Triisopropanolamin, 2-Amino-1-butanol, 2-(2'-Aminoethoxy)-ethanol, 2-Amino-2-methyl-1-propanol und 2-Amino-2-ethyl-1,3-propandiol; wie bereits erwähnt, sind Alkanolamine mit primären Aminogruppen bzw. Gemische derselben mit Alkanolaminen mit sekundären Aminogruppen besonders bevorzugt.

Bevorzugt sind weiterhin auch sekundäre Alkanolamine, die neben einer einzigen Hydroxyalkyl-, Hydroxyalkyl-oxyalkylen-oder Dihydroxyalkylgruppe gemäß den oben für $R^2$ angegebenen Definitionen mit einer Alkylgruppe mit 1 bis 6 Kohlenstoffatomen, z.B. Methyl, Ethyl, n-Propyl, i-Propyl, n-Butyl, i-Butyl, Pentyl, Cyclopentyl, Hexyl oder Cyclohexyl substituiert sind.

Derartige sekundäre Monoalkanol-monoalkylamine sind handelsüblich; typische Vertreter sind Methylhydroxyethyl-amin, n-Butyl-hydroxyethylamin und Cyclohexyl-hydroxyethyl-amin sowie die entsprechend substituierten Hydroxypropylderivate. Die von diesen Monoalkanolmonoalkylaminen abgeleiteten Verbindungen der allgemeinen Formel I weisen zum Teil ausgeprägte fungizide Eigenschaften auf, die die Zugabe anderer Fungizide zur Verbesserung der biostatischen Eigenschaften überflüssig machen.

Beispiele für Alkylengruppen mit 4 bis 6 Kohlenstoffatomen, die den Rest $R^6$ bilden können, sind Butylen-, Pentylen-, Hexylen-, 2-Methyl-propylen-, 2-Methyl-butylen-, 3-Methyl-butylen-, 2,2-Dimethylpropylen- und 2,2-Dimethyl-butylengruppen.

Beispiele für Alkylgruppen mit 12 bis 22 Kohlenstoffatomen, die den Rest $R^7$ bilden können, sind die Dodecyl-, Tridecyl-, Tetradecyl-, Pentadecyl-, Hexadecyl-, Heptadecyl-, Octadecyl-, Nonadecyl-, Eicosyl-, Heneicosyl- und Docosylgruppe.

Die biozide bzw. biostatische Wirkung der erfindungsgemäß verwendeten Aminoester der allgemeinen Formel I erstreckt sich auf Bakterien, Hefen und Pilze. Dabei sind die Grenzen zwischen einer bioziden und einer biostatischen Wirkung fließend. Abhängig von der verwendeten Menge und der Einwirkungsdauer überwiegt entweder die biozide (keimabtötende) oder die biostatische (wachstumshemmende) Wirkung. Verwendet man zusätzlich zu den Aminoestern der Erfindung noch ein Fungizid, treten synergistische Effekte auf, d.h. die Wirkungen verstärken sich gegenseitig. Beispiele für Fungizide sind Pyrithion und dessen Derivate, N-Alkyl- oder N-Aryl-, insbesondere N-Cyclohexyl-, Diazeniumdioxidsalze, z.B. mit Kalium, Aluminium, Zinn oder Kupfer, als Metallkomponente (Ullmanns Encyklopädie der technischen Chemie, 4. Auflage, Band 17, Verlag Chemie, Weinheim 1979, S. 369), Phenole, Kresole, 1,2-Benzisothiazolin-3-on und dessen Derivate sowie 2-Methyl- und 2-Octyl-4-isothiazolin-3-on, wobei halogenfreie Verbindungen bevorzugt sind. Weiterhin werden bevorzugt Fungizide eingesetzt, die wasserlöslich und alkalistabil sind.

Gemäß einer weiteren bevorzugten Ausführungsform der Erfindung werden als Fungizide Pyrithion oder Derivate desselben und/oder N-Alkyl- oder N-Aryl-, insbesondere N-Cyclohexyl-, Diazeniumdioxidsalze, z.B.

mit Kalium, Aluminium, Zinn oder Kupfer, als Metallkomponente verwendet. Pyrithion ist die Kurzbezeichnung für 2-Pyridinthiol-1-oxid, das mit 1-Hydroxy-2-pyridinthion im tautomeren Gleichgewicht steht. Als Derivate des Pyrithions kommen die Ammonium-, Natrium-, Magnesium- und Zinksalze sowie 2,2'-Dithiobis-(pyridin-1,1'-dioxid), das Disulfid des Pyrithions, in Frage. Das Anion des Pyrithions ist unter Umständen mit Schwermetallen fällbar. Dagegen weisen die o.g. N-Alkyl- bzw. N-Aryl-diazeniumdioxidsalze neben fungiziden Eigenschaften auch komplexierende Eigenschaften auf. Daher wird bevorzugt ein Gemisch von Pyrithion bzw. dessen Derivaten und den o.g. N-Alkyldiazeniumdioxidsalzen verwendet. Es können aber auch nur Pyrithion oder Derivate desselben eingesetzt werden, wobei die fungizide Wirkung in Abwesenheit signifikanter Mengen an Schwermetallen erhalten bleibt. Da Kombinationen der genannten Fungizide mit den erfindungsgemäßen Aminoestern synergistische Effekte aufweisen, reichen sehr geringe Mengen derselben für die erfindungsgemäße Verwendung in wasserhaltigen Systemen aus.

Die erfindungsgemäß herstellbaren wasserhaltigen Systeme enthalten 0,05 bis 0,40 Gew.-% der Aminoester der allgemeinen Formel I und 0,0001 bis 0,2 Gew.-%, vorzugsweise 0,001 bis 0,1, insbesondere 0,001 bis 0,02 Gew.-% Fungizide, bezogen auf die Gesamtformulierung.

Es sind Verwendungen in nahezu beliebigen wäßrigen bzw. wasserhaltigen Systemen möglich, z.B. in Metallbearbeitungsflüssigkeiten, Kühlmitteln für Kühlkreisläufe, Reinigern, Hydraulikflüssigkeiten, Kosmetika und Anstrichstoffen. Bei der Verwendung in Kosmetika werden diese bevorzugt nach dem oben beschriebenen Verfahren auf einen pH-Wert im Bereich von 4,5 bis 7,0 eingestellt. Kühlschmierstoffe werden dagegen bevorzugt auf einen pH-Wert im Bereich von 7,5 bis 9,5 eingestellt.

Die Aminoester der allgemeinen Formel I werden insbesondere in Kühlschmierstoffen verwendet.

Kühlschmierstoffe sind wäßrige Flüssigkeiten, die z.B. beim Bohren, Mahlen, Fräsen, Drehen, Schneiden, Sägen, Schleifen, Gewindeschneiden oder beim Walzen oder Ziehen von Metallen zum Kühlen und Schmieren verwendet werden. Diese können nach dem Mineralölanteil in drei Gruppen eingeteilt werden:

a) synthetische Kühlschmierstoffe, die mineralölfrei sind,

b) halbsynthetische Kühlschmierstoffe, die ca. 10 bis 60 Gew.-% Mineralöl enthalten und

c) Kühlschmierstoffe, die ca. 60 bis 80 % Mineralöl enthalten.

Die Kühlschmierstoffe können weiterhin Polyglykole enthalten. Anstelle von Mineralölen können auch natürliche oder synthetische Fettsäureester, z.B. Rüböl oder Esteröle, verwendet werden.

Allen drei Typen von Kühlschmierstoffen können weitere Additive wie Korrosionsinhibitoren, Kupfer-Passivatoren, Antiverschleißmittel, Emulgatoren, Trägerstoffe, Fällungsmittel, Sauerstoffabfänger, Komplexierungsmittel oder schaumverhütende Mittel zugesetzt sein.

Beispiele für Korrosionsinhibitoren sind organische Säuren, deren Salze und Ester, z.B. Benzoesäure, p-tert.-Butylbenzoesäure, Dinatriumsebacat, Triethanolamin-laurat, Isononansäure, das Triethanolaminsalz von p-Toluolsulfonamidocapronsäure, Natrium-N-lauroylsarcosinat oder Nonylphenoxyessigsäure oder Polycarbonsäuren; stickstoffhaltige Substanzen, z.B. Fettsäurealkanolamide, Imidazoline, Oxazoline, Triazole, Triethanolamin, Fettamine, N-Acylsarkosine, oder anorganische Nitrite oder Nitrate; phosphorhaltige Substanzen, z.B. Aminphosphate, Phosphonsäuren, Phosphonate, Phosphonocarbonsäuren, Phosphinocarbonsäuren, oder anorganische Phosphate wie $NaH_2PO_4$, und schwefelhaltige Substanzen, z.B. Salze von Petroleumsulfonaten oder Alkylbenzolsulfonaten, oder heterocyclische Verbindungen, die im Ring ein Schwefelatom oder mehrere enthalten.

Als Kupfer-Passivatoren können z.B. Benztriazole, Methylen-bis-benztriazole wie Natrium-2-mercaptobenztriazol, Thiadiazole, z.B. 2,5-Dimercapto-1,3,4-thiadiazol-Derivate, oder Tolyltriazole dienen.

Antiverschleißmittel können AW(Anti-Wear)- oder EP(Extreme-Pressure)-Additive sein, z.B. Schwefel, Phosphor oder Halogen enthaltende Substanzen, wie sulfurierte Fette und Olefine, Tritolylphosphat, Mono- und Diester der Phosphorsäure, Additionsprodukte von Ethylenoxid und/oder Prolylenoxid an Polyhydroxyverbindungen, die gegebenenfalls partiell mit Fettsäuren verestert sind, Chlorparaffine oder ethoxylierte Phosphatester, wobei chlorfreie Verbindungen bevorzugt sind.

Beispiele für Emulgatoren sind Ethercarbonsäuren, Fettsäurealkanolamide, Natrium-Petroleumsulfonate, Mono- oder Diester oder -ether von Polyethylen-, Polypropylen- oder gemischten Polyethylen/Polypropylenglykolen oder Fettsäureseifen.

Als Trägerstoffe können z.B. Poly(meth)acrylsäure und seine Salze, hydrolysiertes Polyacrylnitril, Polyacrylamid und dessen Copolymere, Ligninsulfonsäure und deren Salze, Stärke und Stärkederivate, Cellulose, Alkylphosphonsäuren, 1-Aminoalkyl-1,1-diphosphonsäuren und ihre Salze, Polymaleinsäuren und andere Polycarbonsäuren, Esteröle, natürliche oder synthetische Fettsäureester, z.B. Rüböl, oder Alkaliphosphate verwendet werden.

Beispiele für Fällungsmittel sind Alkaliphosphate oder Alkalicarbonate.

Beispiele für Sauerstoffabfänger sind Alkalisulfate, Morpholin und Hydrazin.

Die erfindungsgemäßen Aminoester der allgemeinen Formel I weisen selbst komplexierende Eigenschaften auf. Es können aber auch weitere Komplexierungsmittel, z.B. Phosphonsäurederivate, Nitrilotriessigsäure oder Ethylendiamin-tetraessigsäure und deren Salze, zugesetzt werden. Im übrigen weisen auch die gegebenenfalls als Fungizide einzusetzenden N-Alkyl-bzw. N-Aryldiazeniumdioxidsalze komplexierende Eigenschaften auf, worauf bereits hingewiesen wurde.

Beispiele für schaumverhütende Mittel sind Distearylsebacinsäurediamid, Distearyladipinsäurediamid oder Ethylenoxidund/oder Propylenoxid-Additionsprodukte solcher Amide, Fettalkohole und deren Ethylenoxid- und/oder Propylenoxid-Additionsprodukte, natürliche und synthetische Wachse, Silikonverbindungen, Kieselsäurederivate und pyrogenes Siliciumdioxid.

Typische Kühlschmierstoffe im Sinne der Erfindung sind z.B. solche, die

a) Aminoester der allgemeinen Formel I,
b) Fungizide,
c) Wasser,
d) gegebenenfalls Mineralöl,
e) gegebenenfalls Emulgatoren und/oder weitere Hilfsstoffe,
f) gegebenenfalls Korrosionsinhibitoren,

enthalten, wobei die Aminoester in einer Menge von 0,05 bis 0,40 Gew.-% und die Fungizide in einer Menge von 0,0001 bis 0,2 Gew.-%, vorzugsweise 0,001 bis 0,1, insbesondere 0,001 bis 0,02 Gew.-%, bezogen auf die Gesamtmenge des Kühlschmierstoffs, enthalten sind.

Besonders vorteilhaft sind Kühlschmierstoffe, die als Emulgatoren und/oder weitere Hilfsstoffe

a) Ethercarbonsäuren der allgemeinen Formel IV, in der $R^3$, m und q wie oben definiert sind, in Form ihrer Alkanolamide und/oder Alkanolammoniumsalze mit Alkanolaminen der allgemeinen Formel II, in der $R^2$ wie oben definiert ist,

b) Fettsäurealkanolamide auf der Basis geradkettiger oder verzweigter, gesättigter oder ungesättigter Fettsäuren mit 12 bis 22 Kohlenstoffatomen und Aminen der allgemeinen Formel II,

c) Aryl- bzw. Alkylsulfonamidocarbonsäuren der allgemeinen Formel Va bzw. Vb, in denen $R^4$, $R^5$, $R^6$, $R^7$ und $R^8$ wie oben definiert sind, in Form ihrer Alkanolamide und/oder Alkanolammoniumsalze mit Alkanolaminen der allgemeinen Formel II, in der $R^2$ wie oben definiert ist,

d) geradkettige oder verzweigte, ungesättigte oder gesättigte Carbonsäuren mit 5 bis 22, vorzugsweise 5 bis 11, Kohlenstoffatomen zur Einstellung eines pH-Wertes im Bereich von 7,5 bis 9,5, oder

e) geradkettige oder verzweigte Fettalkohole mit 12 bis 18 Kohlenstoffatomen

enthalten.

Weitere besonders vorteilhafte Kühlschmierstoffe enthalten als Fungizide Pyrithion oder Derivate desselben und/oder N-Alkyldiazeniumdioxidsalze.

Die Kühlschmierstoffe können durch Zusammenmischen der Einzelkomponenten hergestellt werden. Falls die Kühlschmierstoffe einen Gehalt an Fettsäurealkanolamiden aufweisen sollen, ist es bevorzugt, die Aminoester der allgemeinen Formel I in der oben erläuterten Weise in Form ihrer Gemische mit den Fettsäurealkanolamiden herzustellen. Dieses Verfahren bietet zudem den Vorteil, daß ausschließlich flüssige Reaktionsgemische erhalten werden, die ohne weitere Maßnahmen, z.B. eine Zerkleinerung oder Auflösung in geeigneten Lösemitteln, weiterverarbeitet werden können.

Die Erfindung wird im folgenden anhand besonders bevorzugter Ausführungsbeispiele näher erläutert.

Die Beispiele 1 bis 12 zeigen die Herstellung von erfindungsgemäß verwendeten Derivaten des 2,4,6-Tris(omega'-carboxypentylamino)-1,3,5-Triazins, im folgenden kurz Triazincarbonsäure genannt, das kommerziell erhältlich oder durch Umsetzung von Cyanursäurechlorid mit dem Natriumsalz der 6-Aminohexansäure gemäß EP-B 0 046 139 erhalten werden kann.

Die Triazincarbonsäure kann als handelsübliches Produkt oder in Form des handelsüblichen wasserhaltigen Produktes verwendet werden. In den folgenden Beispielen wurde ein ca. 50-Gew.% Wasser enthaltendes, festes Produkt eingesetzt.

Beispiel 1

75 g (0,714 mol) Diethanolamin wurden mit 25 g (0,0267 mol) Triazincarbonsäure verrührt. Nach einer Reaktionszeit von mehreren Stunden bei 150 bis 160 °C wurden 10 g Wasser abdestilliert.

Die Endsäurezahl betrug 10 mg KOH/g.

Man erhielt 90 g einer klaren, mittelviskosen Flüssigkeit.

Beispiel 2

75 g (1,230 mol) Monoethanolamin wurden bei 60 °C mit 25 g (0,0267 mol) Triazincarbonsäure verrührt und auf 140 bis 143 °C erhitzt. Nach einer Reaktionszeit von 10 Stunden wurden 18 g Wasser abdestilliert.

Die Endsäurezahl betrug 12 mg KOH/g.

Man erhielt ein weißes, festes Produkt.

Beispiel 3

863 g (9,697 mol; 31,5 mol pro mol Triazincarbonsäure) 2-Amino-1-butanol wurden bei 60 °C mit 287 g (0,307 mol) Triazincarbonsäure verrührt und auf 145 °C erhitzt. Nach einer Reaktionszeit von 20 Stunden wurden 150 g Wasser abdestilliert.

Die Endsäurezahl betrug 10 mg KOH/g.

Man erhielt 1000 g einer klaren, niedrigviskosen Flüssigkeit.

Beispiel 4

375 g (3,571 mol; 26,7 mol pro mol Triazincarbonsäure) 2-(2'-Aminoethoxy)-ethanol wurden bei 60 °C mit 125 g (0,134 mol) Triazincarbonsäure verrührt und auf 145 °C erhitzt. Nach einer Reaktionszeit von 16 Stunden wurden 73 g Wasser abdestilliert.

Die Endsäurezahl betrug 7 mg KOH/g.

Man erhielt 427 g eines weißen, pastösen Produkts.

Beispiel 5

228 g (2,171 mol; 7,1 mol pro mol Triazincarbonsäure) 2-(2'-Aminoethoxy)-ethanol und 627 g (7,045 mol; 23,1 mol pro mol Triazincarbonsäure) 2-Amino-1-butanol wurden bei 60 °C mit 285 g (0,304 mol) Triazincarbonsäure verrührt und auf 145 °C erhitzt. Nach einer Reaktionszeit von 16 Stunden wurden 140 g Wasser abdestilliert.

Die Endsäurezahl betrug 13 mg KOH/g.

Man erhielt 1000 g einer klaren, mittelviskosen Flüssigkeit.

Beispiel 6

833 g (8,424 mol; 31,5 mol pro mol Triazincarbonsäure) AMP 90 wurden auf 60 °C erwärmt, mit 250 g (0,267 mol) Triazincarbonsäure verrührt und auf 140 bis 145 °C erhitzt. Nach einer Reaktionszeit von 20 Stunden wurden 240 g Wasser abdestilliert.

Die Endsäurezahl betrug 15 mg KOH/g.

Man erhielt 843 g eines fast klaren, hochviskosen Produkts.

Beispiel 7

990 g (8,319 mol; 23,6 mol pro mol Triazincarbonsäure) AEPD wurden auf 60 °C erwärmt, mit 330 g (0,353 mol) Triazincarbonsäure verrührt und auf 140 bis 145 °C erhitzt. Nach einer Reaktionszeit von 16 Stunden wurden 320 g Wasser abdestilliert.

Die Endsäurezahl betrug 10 mg KOH/g.

Man erhielt 1000 g einer klaren, hochviskosen Flüssigkeit.

Beispiel 8

375 g (5,000 mol; 37,4 mol pro mol Triazincarbonsäure) Monoisopropanolamin wurden auf 60 °C erwärmt, mit 125 g (0,134 mol) Triazincarbonsäure verrührt und auf 140 °C erhitzt. Nach einer Reaktionszeit von 16 Stunden wurden 74 g Wasser abdestilliert.

Die Endsäurezahl betrug 12 mg KOH/g.

Man erhielt 426 g einer klaren, niedrigviskosen Flüssigkeit.

Beispiel 9

750 g (5,034 mol; 18,9 mol pro mol Triazincarbonsäure) Triethanolamin wurden auf 60 °C erwärmt, mit 250 g (0,267 mol) Triazincarbonsäure verrührt und auf 140 bis 145 °C erhitzt. Nach einer Reaktionszeit von 16 Stunden wurden 130 g Wasser abdestilliert.

Die Endsäurezahl betrug 6 mg KOH/g.

Man erhielt 870 g einer klaren, mittelviskosen Flüssigkeit.

Beispiel 10

1. Stufe

130 g (1,238 mol; 4,8 mol pro mol Triazincarbonsäure) 2-(2'-Aminoethoxy)-ethanol und 370 g (4,157 mol; 16,2 mol pro mol Triazincarbonsäure) 2-Amino-1-butanol wurden mit 190 g (0,674 mol; 2,6 mol pro mol Triazincarbonsäure) Olein bei 145 °C umgesetzt. Nach einer Reaktionszeit von 10 Stunden wurden 12 g Wasser abdestilliert.

Man erhielt 678 g eines flüssigen Produkts mit einer Säurezahl von 7 mg KOH/g.

2. Stufe

678 g der Flüssigkeit der ersten Stufe wurden auf 60 °C erwärmt, mit 240 g (0,256 mol) Triazincarbonsäure verrührt und auf 140 bis 150 °C erhitzt. Nach einer Reaktionszeit von 10 Stunden wurden 138 g Wasser abdestilliert.

Die Endsäurezahl betrug 14 mg KOH/g.

Man erhielt 780 g eines klaren, mittelviskosen Produkts.

Beispiel 11

130 g (1,238 mol) 2-(2'-Aminoethoxy)-ethanol, 370 g (4,157 mol) 2-Amino-1-butanol, 174 g (0,497 mol) Sulfonamidocarbonsäure und 240 g (0,256 mol) Triazincarbonsäure wurden bei 145 °C umgesetzt. Nach einer Reaktionszeit von 12 Stunden wurden 143 g Wasser abdestilliert.

Man erhielt 771 g eines mittelviskosen, klaren, flüssigen Produkts mit einer Endsäurezahl von 25 mg KOH/g.

Beispiel 12

130 g (1,238 mol) 2-(2'-Aminoethoxy)-ethanol, 370 g (4,157 mol) 2-Amino-1-butanol, 106 g (0,148 mol) Ethercarbonsäure und 240 g (0,256 mol) Triazincarbonsäure wurden bei 145 °C umgesetzt. Nach einer Reaktionszeit von 13 Stunden wurden 137 g Wasser abdestilliert.

Man erhielt 709 g eines klaren, mittelviskosen Produkts mit einer Endsäurezahl von 20 mg KOH/g.

Vergleichsbeispiele 1 bis 3 und Beispiele 13 bis 19

Eine Reihe der so hergestellten Aminoester wurde mit Wasser, Spindelöl und weiteren, jeweils angegebenen Zusätzen sowie in einigen Beispielen mit Fungiziden zu Gemischen formuliert, die in einer Verdünnung mit Wasser von 1:20 bis 1:80 Kühlschmierstoffe ergeben.

Weiterhin wurden in den Vergleichsbeispielen 1 und 2 Gemische ohne Biozide und in dem Vergleichsbeispiel 3 ein Gemisch mit einem Borsäurealkanolamin-Kondensationsprodukt als biozides Mittel formuliert.

Angaben in % beziehen sich im folgenden immer auf Gewichtsteile.

Die Angaben in der Spalte "Erl." beziehen sich auf die Erläuterungen der Tabelle 1. Sämtliche in der Tabelle 1 aufgeführten Chemikalien sind im Handel erhältlich.

Tabelle 1: E R L Ä U T E R U N G E N

| | | |
|---|---|---|
| 1 | | Tallöldestillat mit 25-30% Harz (Säurezahl 155-190) |
| 2 | a) | Isononansäure |
| | b) | 2,2-Dimethyl-octansäure |
| 3 | | Spindelöl, Viskosität: 22 $mm^2/s$ bei 40°C |
| 4 | a) | Umsetzungsprodukt von 1 mol Chloressigsäure mit einem Kondensationsprodukt von 1 mol eines technischen Oleylalkohols mit 10 mol Ethylenoxid (Ethercarbonsäure) |
| | b) | Umsetzungsprodukt von 1 mol Chloressigsäure mit einem Kondensationsprodukt von 1 mol $C_9$- bis $C_{13}$-Oxoalkohole mit 3 mol Ethylenoxid und 2 mol Propylenoxid (Ethercarbonsäure) |
| 5 | a) | technischer Oleylalkohol (ca. 90%-ig, Jodzahl ca. 95) |
| | b) | 2-Hexyl-decanol |
| 6 | a) | Kondensationsprodukt von 1 mol eines technischen Gemisches von Oleyl- und Cetylalkohol mit 5 mol Ethylenoxid |
| | b) | Fettalkohol-polyglykolether (Emulsogen® LP) |
| 7 | a) | Kondensationsprodukt von 40 Gewichtsteilen Diethanolamin mit 60 Gewichtsteilen Olein |
| | b) | wie a) unter Zusatz von 20% Ethanolamin, bezogen auf die Gesamtmenge von Kondensationsprodukt und Ethanolamin |
| 8 | a) | Diethylenglykol |
| | b) | Butyldiglykol |
| | c) | Butylglykol |
| 9 | a) | Natriumpetroleumsulfonat mit einem Molekulargewicht von ca. 460 |
| | b) | Natriumalkylbenzolsulfonat mit einem Molekulargewicht von ca. 350 |
| 10 | | 50 %-ige Kalilauge |

Fungizides Gemisch aus

10 % Natriumsalz des Pyrithions

10 % N-(Cylohexyl-diazeniumdioxid)-Kalium-Hydrat in Form einer 30 %-igen wäßrigen Lösung

10 % Propylenglykol

70 % demineralisiertem Wasser

Kondensationsprodukt von 1 mol Borsäure mit 3 mol Ethanolamin

Arylsulfonamidocarbonsäure mit einem Molekulargewicht von ca. 350 (Hostacor$^R$ H flüssig; Säuregehalt ca. 90 %-ig, Rest Lösevermittler).

| Vergleichsbeispiel 1 | Erl. |
|---|---|
| 7 % Fettsäuren | 1 |
| 2 % Sulfonate | 9b) |
| 5 % Fettsäurealkanolamide | 7b) |
| 2 % Hilfsstoffe | 8a) |
| 1 % Hilfsstoffe | 10 |
| 83 % Spindelöl | 3 |

| Vergleichsbeispiel 2 | Erl. |
|---|---|
| 8 % Fettsäuren | 1 |
| 17 % Sulfonate | 9a) |
| 4 % Fettsäurealkanolamide | 7a) |
| 3 % Hilfsstoffe | 8c) |
| 2 % Hilfsstoffe | 10 |
| 36 % Spindelöl | 3 |
| 30 % Wasser | |

| Vergleichsbeispiel 3 | Erl. |
|---|---|
| 20 % Borsäureprodukt | 12 |
| 10 % Fettsäuren | 1 |
| 10 % Fettsäurealkanolamide | 7a) |
| 10 % Hilfsstoffe | 8b) |
| 20 % Spindelöl | 3 |
| 30 % Wasser | |

| Beispiel 13 | Erl. |
|---|---|
| 25 % Beispiel 1 | |
| 31 % Spindelöl | 3 |
| 10 % Fettsäuren | 1 |
| 5 % Fettsäuren | 2b) |
| 4 % Ethercarbonsäuren | 4b) |
| 6 % Fettalkohole | 5a) |
| 14 % Wasser | |
| 5 % nichtionische Emulgatoren | 6a) |

| Beispiel 14 | Erl. |
|---|---|
| 25 % Beispiel 2 | |
| 31 % Spindelöl | 3 |
| 10 % Fettsäuren | 1 |
| 5 % Fettsäuren | 2b) |
| 4 % Ethercarbonsäuren | 4b) |
| 6 % Fettalkohole | 5a) |
| 5 % nichtionische Emulgatoren | 6a) |
| 14 % Wasser | |

| Beispiel 15 | Erl. |
|---|---|
| 22 % Beispiel 3 | |
| 11 % Fettsäuren | 2a) |
| 17 % Fettsäuren | 1 |
| 6 % Ethercarbonsäuren | 4a) |
| 9 % Fettsäurealkanolamide | 7a) |
| 4 % Fettalkohole | 5a) |
| 22 % Spindelöl | 3 |
| 8 % Wasser | |
| 1 % Fungizide | 11 |

| Beispiel 16 | Erl. |
|---|---|
| 21 % Beispiel 5 | |
| 21 % Fettsäuren | 1 |
| 11 % Fettsäuren | 2a) |
| 7 % Ethercarbonsäuren | 4a) |
| 5 % Fettalkohole | 5b) |
| 20 % Spindelöl | 3 |
| 13 % Wasser | |
| 2 % Fungizide | 11 |

| Beispiel 17 | Erl. |
| --- | --- |
| 25 % Beispiel 7 | |
| 31 % Spindelöl | 3 |
| 10 % Fettsäuren | 1 |
| 3 % Fettsäuren | 2a) |
| 8 % Fettalkohole | 5a) |
| 5 % nichtionische Emulgatoren | 6a) |
| 4 % Ethercarbonsäuren | 4b) |
| 1 % Fungizide | 11 |
| 13 % Wasser | |

| Beispiel 18 | Erl. |
| --- | --- |
| 25 % Beispiel 8 | |
| 31 % Spindelöl | 3 |
| 10 % Fettsäuren | 1 |
| 5 % Fettsäuren | 2b) |
| 4 % Ethercarbonsäuren | 4b) |
| 6 % Fettalkohole | 5a) |
| 5 % nichtionische Emulgatoren | 6a) |
| 1 % Fungizide | 11 |
| 13 % Wasser | |

| Beispiel 19 | Erl. |
| --- | --- |
| 19 % Beispiel 10 | |
| 29 % Fettsäuren | 1 |
| 29 % Spindelöl | 3 |
| 5 % Hilfsstoffe | 8b) |
| 3 % nichtionische Emulgatoren | 6b) |
| 1 % Fungizide | 11 |
| 14 % Wasser | |

| Beispiel 20 | Erl. |
| --- | --- |
| 23 % Beispiel 11 | |
| 28 % Fettsäuren | 1 |
| 25 % Spindelöl | 3 |
| 5 % nichtionische Emulgatoren | 6b) |
| 2 % Fettalkohole | 5b) |
| 2 % Hilfsstoffe | 8b) |
| 1 % Fungizide | 11 |
| 14 % Wasser | |

| Beispiel 21 | Erl. |
|---|---|
| 23 % Beispiel 12 | |
| 28 % Fettsäuren | 1 |
| 1 % Fettalkohole | 5a) |
| 4 % nichtionische Emulgatoren | 6a) |
| 4 % Hilfsstoffe | 8b) |
| 1 % Fungizide | 11 |
| 27 % Spindelöl | 3 |
| 12 % Wasser | |

Mikrobiologisches Testverfahren

Es wurde ein selbstentwickelter Impfzyklentest durchgeführt. Dazu wurden folgende Verdünnungen der Formulierungen der Vergleichsbeispiele 1 bis 3 und der Beispiele 26 bis 42 mit Hamburger Stadtwasser angesetzt: 1,25 %, 2,5 % und 5,0 % (entspricht 1:80, 1:40 und 1:20).

Die Proben wurden mit einer konzentrierten Mischkeimflora mehrfach beimpft. Die Keimflora enthielt Bakterien, Hefen und Pilze aus laufenden Emulsionssystemen unterschiedlicher Herkunft. Ihre Gesamtkeimzahl betrug ca. $10^7$ Keime/ml.

Die Menge an Mischkeimflora bei der Beimpfung der Proben entsprach der sechsfachen Menge, die nach DAB 9 (Deutsches Arzneibuch) vorgeschlagen wird. Es wurden je 100 ml Probe 6 ml Keimflora verwendet.

Die Proben wurden (in Anlehnung an K.H. Wallhäußer; Praxis der Sterilisation-Desinfektion-Konservierung-Keimidentifizierung, 4. Auflage, Georg Thieme Verlag, Stuttgart 1988) nach diesem Verfahren wiederholt beimpft (maximal 6 Beimpfungen), bis eine antimikrobielle Wirkung nicht mehr feststellbar war. Erfahrungsgemäß entsprach 1 Beimpfung 3 Impfzyklen nach der DAB-9/Wallhäuser-Methode.

Dieses Verfahren hat folgende Vorteile:

1. Es wird eine Mischkeimflora eingesetzt, wie sie in der Praxis vorkommt.
2. Die Proben werden mehrfach einer massiven Keimbelastung ausgesetzt.
3. Die Methode ist schnell und damit industriegerecht. Im Vergleich zur herkömmlichen Methode, die oft mehrere Monate dauert, liegen die Ergebnisse in max. 8 Wochen vor, wenn sie nicht wiederholt werden müssen.
4. Aus den Ergebnissen lassen sich Rückschlüsse ziehen auf die Standzeiten der Gebrauchsemulsionen in den Zentralsystemen.

Die Einwirkzeit der Mikroorganismen auf die Proben betrug ca. 1 Woche. Nach dieser Zeit wurden die Proben auf je zwei Spezialnährböden ausgestrichen und bebrütet. Anschließend wurde unter dem Mikroskop die Kolonienzahl ermittelt und daraus die Keimzahl pro ml Probe bestimmt. Die Anzahl der Impfzyklen, nach denen ein erster Keimbefall zu beobachten ist, ist in Tabelle 2 gezeigt. Diese stellt ein Maß für die Wirksamkeit der Biozide in den jeweiligen Proben dar. Als besonders wirksam erwiesen sich die Formulierungen der Beispiele 13, 14, 15 bzw. 18 auf der Basis der Verbindungen der Beispiele 1, 2, 3 bzw. 8. Beispiel 14 zeigt sogar ohne Zusatz von Pyrithion oder dessen Derivaten eine fungizide Wirkung.

Tabelle 2

| Mikrobiologische Ergebnisse | | | | | | | | | | | | |
|---|---|---|---|---|---|---|---|---|---|---|---|---|
| Verdünnung % | 1,25 | | | | 2,5 | | | | 5 | | | |
| Beispiel | B | H | P | IZ | B | H | P | IZ | B | H | P | IZ |
| Vergleichsbsp. 1 | | | | | | | | | + + + | + + | 0 | 3 |
| Vergleichsbsp. 2 | | | | | | | | | + + + | + + | 0 | 3 |
| Vergleichsbsp. 3 | | | | | | | | | 0 | 0 | + + + | 18 |
| 13 | + + + | + + + | 0 | 3 | + + + | 0 | + + | 12 | 0 | 0 | 0 | 18 |
| 14 | + + + | + + | 0 | 9 | + + | + + | 0 | 12 | + | 0 | 0 | 18 |
| 15 | + + | + + | 0 | 3 | + + | + + | 0 | 12 | 0 | 0 | 0 | 18 |
| 16 | + + + | 0 | 0 | 3 | + + + | 0 | + + + | 9 | 0 | 0 | 0 | 18 |
| 17 | + + | + + | 0 | 3 | + | 0 | 0 | 6 | + + | + | 0 | 9 |
| 18 | + + + | + + | 0 | 6 | 0 | 0 | 0 | 18 | 0 | 0 | 0 | 18 |
| 19 | + + + | + + | 0 | 3 | + + | + + | 0 | 3 | + | + | 0 | 15 |

+ + + = starker Befall - Keimzahl/ml > $10^4$ B = Bakterien
+ + = mittlerer Befall - Keimzahl/ml $10^3$-$10^4$ H = Hefen
+ = schwacher Befall - Keimzahl/ml < $10^3$ P = Pilze
0 = kein Befall
IZ = erster Keimbefall nach x Impfzyklen

## Patentansprüche

**1.** 1,3,5-Triazin-2,4,6-tris-alkylaminocarbonsäureaminoester der allgemeinen Formel

1,3,5-Triazin-2,4,6-tris[NH-$(CH_2)_n$-CO-O-$R^1$] (I)

in der
n eine Zahl im Bereich von 4 bis 11 bedeutet und
$R^1$ einen Rest eines Alkanolamins der allgemeinen Formel

$(R^2)_3N$ (II)

in der
mindestens eine der Gruppen $R^2$
a) eine Hydroxyalkylgruppe mit 2 bis 4 Kohlenstoffatomen,
b) eine Hydroxyalkyl-oxyalkylengruppe mit 4 bis 6 Kohlenstoffatomen oder
c) eine Dihydroxyalkylgruppe mit 3 bis 6 Kohlenstoffatomen
und, wenn weniger als drei der Gruppen $R^2$ die vorstehende Bedeutung aufweisen, die übrigen Gruppen $R^2$ Wasserstoff sind, oder eine der Gruppen $R^2$ die vorstehend genannten Bedeutungen aufweist sowie die zweite eine Alkylgruppe mit 1 bis 6 Kohlenstoffatomen und die dritte Wasserstoff ist, bedeutet.

**2.** 1,3,5-Triazin-2,4,6-tris-alkylaminocarbonsäureaminoester der allgemeinen Formel I nach Anspruch 1, in der n die Zahl 5 bedeutet und $R^1$ wie oben definiert ist.

**3.** 1,3,5-Triazin-2,4,6-tris-alkylaminocarbonsäureaminoester der allgemeinen Formel I nach Anspruch 1 oder 2, in der n wie oben definiert und der Rest $R^1$ ausschließlich von primären und sekundären Alkanolaminen der allgemeinen Formel II abgeleitet ist.

**4.** Verfahren zur Herstellung von 1,3,5-Triazin-2,4,6-trisalkylaminocarbonsäureaminoester gemäß mindestens einem der Ansprüche 1 bis 3, dadurch gekennzeichnet, daß man 1,3,5-Triazin-2,4,6-tris-alkylaminocarbonsäuren der allgemeinen Formel

1,3,5-Triazin-2,4,6-tris[-NH-$(CH_2)_n$-COOH] (III)

in der n wie oben definiert ist, in an sich bekannter Weise mit Alkanolaminen der allgemeinen Formel II, in der $R^2$ wie oben definiert ist, umsetzt.

**5.** Verfahren zur Herstellung eines bioziden bzw. biostatischen Gemisches von Monocarbonsäurealkanolamiden und 1,3,5-Triazin-2,4,6-tris-alkylaminocarbonsäureaminoestern nach Anspruch 1 oder 2 sowie gegebenenfalls Alkanolammoniumsalzen der Monocarbonsäuren und/oder der 1,3,5-Triazin-2,4,6-tris-alkylaminocarbonsäuren, dadurch gekennzeichnet, daß man als Monocarbonsäuren geradkettige oder verzweigte, gesättigte oder ungesättigte Fettsäuren mit 3 bis 22, insbesondere 12 bis 22 Kohlenstoffatomen,
Ethercarbonsäuren der allgemeinen Formel

$R^3$-(O-$C_mH_{2m}$)$_q$-O-$CH_2$-COOH (IV)

in der
$R^3$ eine geradkettige oder verzweigte Alkyl- oder Alkenylgruppe mit 9 bis 18 Kohlenstoffatomen, bedeutet, Arylsulfonamidocarbonsäuren der allgemeinen Formel

($R^4$)Aryl-$SO_2$-N($R^5$)-$R^6$-COOH (Va)

in der
$R^4$ Wasserstoff oder eine Methyl- oder Ethylgruppe oder mehrere, $R^5$ Wasserstoff, eine Methyl-, Ethyl-, beta-Cyanoethyl- oder Hydroxymethylgruppe, $R^6$ eine Alkylengruppe mit 4 bis 6 Kohlenstoffatomen und Aryl einen Phenyl-, Naphthyl- oder Antracenylrest bedeuten,
und/oder Alkylsulfonamidocarbonsäuren der allgemeinen Formel

$R^7$-$SO_2$-$NR^8$-$CH_2$-COOH (Vb)

in der
$R^7$ eine geradkettige oder verzweigte Alkylgruppe mit 12 bis 22 Kohlenstoffatomen und $R^8$ Wasserstoff oder die Gruppe -$CH_2$COOH bedeuten,
einsetzt und diese sowie die 1,3,5-Triazin-2,4,6-tris-alkylaminocarbonsäurender allgemeinen Formel

1,3,5-Triazin-2,4,6-tris[-NH-$(CH_2)_n$-COOH] (III)

in der n wie oben definiert ist, mit den Alkanolaminen der allgemeinen Formel II, in der $R^2$ wie oben definiert ist, umsetzt.

**6.** Verfahren nach Anspruch 4 oder 5, dadurch gekennzeichnet, daß man primäre Alkanolamine oder Gemische aus primären und sekundären Alkanolaminen der allgemeinen Formel II, in der $R^2$ wie oben definiert ist, verwendet.

**7.** Verfahren nach mindestens einem der Ansprüche 4 bis 6, dadurch gekennzeichnet, daß man die Umsetzungen bei einer Temperatur im Bereich von 100 bis 180 °C, insbesondere 130 bis 180 °C, durchführt.

**8.** Verfahren nach mindestens einem der Ansprüche 5 bis 7, dadurch gekennzeichnet, daß man pro Mol der 1,3,5-Triazin-2,4,6-tris-alkylaminocarbonsäuren 10 bis 50, insbesondere 10 bis 30, Mol der Alkanolamine und 0,5 bis 5 Mol der Monocarbonsäuren umsetzt.

**9.** Verfahren nach mindestens einem der Ansprüche 5 bis 8, dadurch gekennzeichnet, daß man die geradkettigen oder verzweigten, gesättigten oder ungesättigten Fettsäuren mit 3 bis 22, insbesondere 12 bis 22 Kohlenstoffatomen, in einer ersten Stufe mit den Alkanolaminen umsetzt, gefolgt von einer Zugabe und Umsetzung der 1,3,5-Triazin-2,4,6-tris-alkylaminocarbonsäuren in einer zweiten Stufe.

**10.** Verfahren nach mindestens einem der Ansprüche 4 bis 9, dadurch gekennzeichnet, daß man in dem Reaktionsgemisch gegebenenfalls enthaltenes überschüssiges Alkanolamin der allgemeinen Formel II zur Einstellung eines pH-Wertes im Bereich von 4,5 bis 9,5 mit Fettsäuren mit 3 bis 22, vorzugsweise 3 bis 11, Kohlenstoffatomen, Ethercarbonsäuren der allgemeinen Formel IV und/oder Aryl- bzw. Alkylsulfonamidocarbonsäuren der allgemeinen Formel Va bzw. Vb umsetzt.

**11.** Verfahren nach mindestens einem der Ansprüche 4 bis 10, dadurch gekennzeichnet, daß man dem Reaktionsgemisch nach der Umsetzung Fungizide zusetzt.

**12.** Verfahren nach mindestens einem der Ansprüche 4 bis 11, dadurch gekennzeichnet, daß man die Fungizide in einer Menge von 1 Gewichtsteil auf 10-100 Gewichtsteile der in dem bioziden bzw. biostatischen Gemisch enthaltenen 1,3,5-Triazin-2,4,6-tris-alkylaminocarbonsäureaminoester der allgemeinen Formel I, in der $R^1$ und n wie oben definiert sind, zusetzt.

**13.** Verfahren nach mindestens einem der Ansprüche 4 bis 11, dadurch gekennzeichnet, daß man als Fungizide Pyrithion oder Derivate desselben und/oder N-Alkyl-diazeniumdioxidsalze verwendet.

**Claims**

**1.** 1,3,5-Triazine-2,4,6-tris-alkylaminocarboxylic acid amino esters of the general formula

1,3,5-triazine-2,4,6-tris[NH-$(CH_2)_n$-CO-O-$R^1$] (I)

in which
n denotes a number in the range from 4 to 11 and $R^1$ denotes a radical of an alkanolamine of the general formula

$(R^2)_3N$ (II)

in which
at least one of the groups $R^2$ is
a) a hydroxyalkyl group having 2 to 4 carbon atoms,
b) a hydroxyalkyl-oxyalkylene group having 4 to 6 carbon atoms or
c) a dihydroxyalkyl group having 3 to 6 carbon atoms and, if less than three of the groups $R^2$ have the above meaning, the other groups $R^2$ are hydrogen, or one of the groups $R^2$ has the abovementioned meanings and the second is an alkyl group having 1 to 6 carbon atoms and the third is hydrogen.

**2.** 1,3,5-Triazine-2,4,6-tris-alkylaminocarboxylic acid amino esters of the general formula I according to Claim 1, in which n denotes the number 5 and $R^1$ is as defined above.

**3.** 1,3,5-Triazine-2,4,6-tris-alkylaminocarboxylic acid amino esters of the general formula I according to Claim 1 or 2, in which n is as defined above and the radical $R^1$ is derived exclusively from primary and secondary alkanolamines of the general formula II.

**4.** Process for the preparation of 1,3,5-triazine-2,4,6-tris-alkylaminocarboxylicacid amino esters according to at least one of Claims 1 to 3, characterized in that 1,3,5-triazine-2,4,6-tris-alkylaminocarboxylic acids of the general formula

1,3,5-triazine-2,4,6-tris[-NH-$(CH_2)_n$-COOH] (III)

in which n is as defined above, are reacted with alkanol-amines of the general formula II, in which $R^2$ is as defined above, in a manner which is known per se.

**5.** Process for the preparation of a biocidal or biostatic mixture of monocarboxylic acid alkanolamides and 1,3,5-triazine-2,4,6-tris-alkylaminocarboxylic acid amino esters according to Claim 1 or 2 and if appropriate alkanolammonium salts of monocarboxylic acids and/or 1,3,5-triazine-2,4,6-tris-alkylaminocarboxylic acids, characterized in that straight-chain or branched, saturated or unsaturated

fatty acids having 3 to 22, in particular 12 to 22, carbon atoms,
ether-carboxylic acids of the general formula

$R^3$-(O-$C_mH_{2m}$)$_q$-O-$CH_2$-COOH (IV)

in which
$R^3$ denotes a straight-chain or branched alkyl or alkenyl group having 9 to 18 carbon atoms, arylsulphonamidocarboxylic acids of the general formula

($R^4$)aryl-$SO_2$-N($R^5$)-$R^6$-COOH (Va)

in which
$R^4$ denotes hydrogen or a methyl or ethyl group or several groups, $R^5$ denotes hydrogen or a methyl, ethyl, betacyanoethyl or hydroxymethyl group, $R^6$ denotes an alkylene group having 4 to 6 carbon atoms and aryl denotes a phenyl, naphthyl or anthracenyl radical, and/or alkylsulphonamidocarboxylic acids of the general formula

$R^7$-$SO_2$-$NR^8$-$CH_2$,-COOH (Vb)

in which
$R^7$ denotes a straight-chain or branched alkyl group having 12 to 22 carbon atoms and $R^8$ denotes hydrogen or the group -$CH_2$COOH,
are employed as the monocarboxylic acids, and these and the 1,3,5-triazine-2,4,6-tris-alkylaminocarboxylic acids of the general formula

1,3,5-triazine-2,4,6-tris[-NH-($CH_2$)$_n$-COOH] (III)

in which n is as defined above, are reacted with the alkanolamines of the general formula II, in which $R^2$ is as defined above.

**6.** Process according to Claim 4 or 5, characterized in that primary alkanolamines or mixtures of primary and secondary alkanolamines of the general formula II, in which $R^2$ is as defined above, are used.

**7.** Process according to at least one of Claims 4 to 6, characterized in that the reactions are carried out at a temperature in the range from 100 to 180 °C, in particular 130 to 180 °C.

**8.** Process according to at least one of Claims 5 to 7, characterized in that 10 to 50, in particular 10 to 30, mol of the alkanolamines and 0.5 to 5 mol of the monocarboxylic acids are reacted per mol of the 1,3,5-triazine-2,4,6-tris-alkylaminocarboxylic acids.

**9.** Process according to at least one of Claims 5 to 8, characterized in that the straight-chain or branched, saturated or unsaturated fatty acids having 3 to 22, in particular 12 to 22, carbon atoms are reacted with the alkanolamines in a first stage, followed by addition and reaction of the 1,3,5-triazine-2,4,6-tris-alkylaminocarboxylic acids in a second stage.

**10.** Process according to at least one of Claims 4 to 9, characterized in that excess alkanolamine of the general formula II contained, if appropriate, in the reaction mixture is reacted with fatty acids having 3 to 22, preferably 3 to 11, carbon atoms, ether-carboxylic acids of the general formula IV and/or aryl- or alkylsulphonamidocarboxylic acids of the general formula Va or Vb to establish a pH in the range from 4.5 to 9.5.

**11.** Process according to at least one of Claims 4 to 10, characterized in that fungicides are added to the reaction mixture after the reaction.

**12.** Process according to at least one of Claims 4 to 11, characterized in that the fungicides are added in an amount of 1 part by weight per 10-100 parts by weight of the 1,3,5-triazine-2,4,6-tris-alkylaminocarboxylic acid amino esters of the general formula I, in which $R^1$ and n are as defined above, contained in the biocidal or biostatic mixture.

13. Process according to at least one of Claims 4 to 11 [sic], characterized in that pyrithione or derivatives thereof and/or N-alkyl-diazenium dioxide salts are used as the fungicides.

**Revendications**

1. Aminoesters d'acides 1,3,5-triazine-2,4,6-tris-alkylaminocarboniques de la formule générale

$$1,3,5\text{-triazine-}2,4,6\text{-tris-}[NH\text{-}(CH_2)_n\text{-}CO\text{-}O\text{-}R^1] \quad (I)$$

dans laquelle
n désigne un nombre dans le domaine de 4 à 11 et $R^1$ un résidu d'une alcanolamine de la formule générale

$$(R^2)_3N \quad (II)$$

dans laquelle au moins l'un des groupes $R^2$ est
a) un groupe hydroxyalkyle avec 2 à 4 atomes de carbone,
b) un groupe hydroxyalkyl-oxyalkylène avec 4 à 6 atomes de carbone ou
c) un groupe dihydroxyalkyle avec 3 à 6 atomes de carbone
et, si moins de trois des groupes $R^2$ présentent la signification précédente, les autres groupes $R^2$ sont l'hydrogène, ou un des groupes $R^2$ qui présente la signification donnée précédemment et le deuxième est un groupe alkyle avec 1 à 6 atomes de carbone et le troisième est l'hydrogène.

2. Aminoesters d'acides 1,3,5-triazine-2,4,6-tris-alkylaminocarboniques de la formule générale I selon la revendication 1, dans laquelle n représente le nombre 5 et $R^1$ est défini comme ci-dessus.

3. Aminoesters d'acides 1,3,5-triazine-2,4,6-tris-alkylaminocarboniques de la formule générale I selon la revendication 1 ou 2, dans laquelle n est défini comme ci-dessus et le résidu $R^1$ est dérivé exclusivement d'alcanolamines primaires et secondaires de la formule générale II.

4. Procédé de fabrication d'aminoesters d'acides 1,3,5-triazine-2,4,6-tris-alkylaminocarboniques selon l'une quelconque des revendications 1 à 3, caractérisé en ce qu'on convertit d'une manière connue en soi des acides 1,3,5-triazine-2,4,6-tris-alkylaminocarboniques de la formule générale

$$1,3,5\text{-triazine-}2,4,6\text{-tris-}[\text{-}NH\text{-}(CH_2)_n\text{-}COOH] \quad (III)$$

dans laquelle n est défini comme ci-dessus, avec des alcanolamines de la formule générale II, dans laquelle $R^2$ est défini comme ci-dessus.

5. Procédé de fabrication d'un mélange biocide ou biostatique d'alcanolamides d'acides monocarboniques et d'aminoesters d'acides 1,3,5-triazine-2,4,6-tris-alkylamino-carboniques selon la revendication 1 ou 2, ainsi que, le cas échéant, de sels d'alcanolammonium des acides monocarboniques et/ou des acides 1,3,5-triazine-2,4,6-tris-alkylaminocarboniques, caractérisé en ce qu'on utilise, comme acides monocarboniques, des acides gras à chaînes droites ou ramifiées, saturés ou insaturés, avec 3 à 22, en particulier 12 à 22, atomes de carbone, des acides éthercarboniques de la formule générale

$$R^3\text{-}(O\text{-}C_mH_{2m})_q\text{-}O\text{-}CH_2\text{-}COOH \quad (IV)$$

dans laquelle
$R^3$ désigne un groupe alkyle ou alcényle à chaîne droite ou ramifiée avec 9 à 18 atomes de carbone, des acides arylsulfonamidocarboniques de la formule générale

$$(R^4)aryl\text{-}SO_2\text{-}N\text{-}(R^5)\text{-}R^6\text{-}COOH \quad (Va)$$

dans laquelle
$R^4$ désigne l'hydrogène ou un groupe méthyle ou éthyle ou plusieurs, $R^5$ l'hydrogène, un groupe méthyle, éthyle, bétacyanoéthyle ou hydroxyméthyle, R6 un groupe alcylène avec 4 à 6 atomes de carbone, et aryle un résidu phényle, naphthyle ou antracényle,

et/ou des acides alkylsulfonamidocarboniques de la formule générale

$R^7$-$SO_2$-$NR^8$-$CH_2$-COOH (Vb)

dans laquelle
$R^7$ désigne un groupe alkyle à chaîne droite ou ramifiée avec 12 à 22 atomes de carbone et $R^8$ l'hydrogène ou le groupe -$CH_2$COOH,
et qu'on convertit ceux-ci, ainsi que les acides 1,3,5-triazine-2,4,6-tris-alkylaminocarboniques de la formule générale

1,3,5-triazine-2,4,6-tris-[-NH-$(CH_2)_n$-COOH] (III)

dans laquelle n est défini comme ci-dessus,
avec les alcanolamines de la formule générale II, dans laquelle $R^2$ est défini comme ci-dessus.

**6.** Procédé selon la revendication 4 ou 5, caractérisé en ce qu'on utilise des alcanolamines primaires ou des mélanges d'alcanolamines primaires et secondaires de la formule générale II, dans laquelle $R^2$ est défini comme ci-dessus.

**7.** Procédé selon l'une quelconque des revendications 4 à 6, caractérisé en ce qu'on effectue les conversions à une température dans le domaine de 100 à 180 °C, en particulier 130 à 180 °C.

**8.** Procédé selon l'une quelconque des revendications 5 à 7, caractérisé en ce que, par mole d'acides 1,3,5-triazine-2,4,6-tris-alkylaminocarbiques, on convertit 10 à 50, en particulier 10 à 30, moles d'alcanolamines et 0,5 à 5 moles d'acides monocarboniques.

**9.** Procédé selon l'une quelconque des revendications 5 à 8, caractérisé en ce qu'on convertit les acides gras à chaînes droites ou ramifiées, saturés ou insaturés, avec 3 à 22, en particulier 12 à 22, atomes de carbone, avec les alcanolamines dans une première étape, suivie d'un ajout et d'une conversion des acides 1,3,5-triazine-2,4,6-tris-alkylaminocarboniques dans une deuxième étape.

**10.** Procédé selon l'une quelconque des revendications 4 à 9, caractérisé en ce qu'on convertit l'alcanolamine de la formule générale II éventuellement présente en excès dans le mélange réactionnel pour ajuster une valeur de pH dans le domaine de 4,5 à 9,5, avec des acides gras avec 3 à 22, de préférence 3 à 11, atomes de carbone, des acides éthercarboniques de la formule générale IV et/ou des acides aryl- ou alkylsulfonamidocarboniques de la formule générale Va, respectivement Vb.

**11.** Procédé selon l'une quelconque des revendications 4 à 10, caractérisé en ce qu'on ajoute des fongicides au mélange réactionnel après la conversion.

**12.** Procédé selon l'une quelconque des revendications 4 à 11, caractérisé en ce qu'on ajoute les fongicides dans une quantité de 1 partie en poids pour 100 parties en poids des aminoesters d'acides 1,3,5-triazine-2,4,6-tris-alkylamino-carboniques, contenus dans le mélange biocide ou biostatique, de la formule générale I, dans laquelle $R^1$ et n sont définis comme ci-dessus.

**13.** Procédé selon l'une quelconque des revendications 4 à 11, caractérisé en ce qu'on utilise comme fongicide du pyrithion ou des dérivés de celui-ci et/ou des sels de dioxyde de N-alkyl-diazénium.